# EUROPEAN PATENT APPLICATION

(11) **EP 3 756 629 A1**
(43) Date of publication of application: **30.12.2020**
(21) Application number: 20182684.9
(22) Date of filing: 26.06.2020
(51) Int. Cl.: A61F 5/44, A61F 5/445, A61F 5/451

(54) **ENTERIC FISTULA, RECTOVAGINAL FISTULA, AND OSTOMY EFFLUENT CONTAINMENT SYSTEM, AND DEVICES AND METHODS THEREOF**

(30) Priority: 26.06.2019 US 201916453315
(71) Applicant: Obst, Andrew Thomas, Scandia, MN 55073 (US); Obst, Maryanne Ruth, Scandia, MN 55073 (US)
(72) Inventor: Obst, Andrew Thomas, Scandia, MN 55073 (US); Obst, Maryanne Ruth, Scandia, MN 55073 (US)
(74) Representative: Barker Brettell LLP

(57) **Abstract**

A skin-safe effluent containment system (100) that may be used to contain fluid leakage from openings in the body of a patient and will adhere to skin in any condition: healthy or injured, wet or dry, smooth, scarred, or uneven. Fluid leakage can occur from natural orifices such as the anus and vagina, spontaneously formed enteric fistulas, rectovaginal fistulas, and surgical openings such as ileostomy and colostomy stomas. The system (100) includes a vacuum-adherent pouch device (10), comprised of a pouch (40) connected to a porous foam ring (46) which has an air-permeable contact layer (56) on its bottom surface and an air seal on its top surface. The inner and outer perimeters of the foam ring (46) are enclosed and sealed with skin-adhesive materials. When placed on the patient, negative pressure (vacuum) is applied to the foam ring (46) so that the foam collapses and compresses the vacuum-adherent pouch device (10) down against the patient's skin. Negative pressure in combination with the adhesive materials on the perimeters of the foam ring (46) forms a fluid barrier around the opening in the body of the patient so that leakage can be directed into the pouch (40) and drawn away to a separate effluent container (16).

## Description

### TECHNICAL FIELD

This disclosure relates to medical devices for use with bodily fluid management systems, and, in particular, to bodily fluid containment systems. This application is related to U.S. Pat. App. Serial No. 13/750,154, filed January 25, 2013 and entitled "Devices and Methods for Treatment of Fistulas and Complex Wounds," now U.S. Pat. No. 9,078,990, U.S. Pat. App. Serial No. 14/931,204, filed November 3, 2015 and entitled "Containment Devices for Treatment of Intestinal Fistulas and Complex Wounds," and U.S. Pat. App. Serial No. 16/275,029, filed February 13, 2019 and entitled "Fluid Management Device for Medical Tubes and Drainage Incisions," all of which are hereby incorporated by reference in their entireties.

### BACKGROUND

Uncontrolled, high-volume leakage of intestinal effluent and fecal matter is a daily challenge for many medical patients with enterocutaneous fistulas (ECF), enteroatmospheric fistulas (EAF), rectovaginal fistulas (RVF), and ostomy stomas. Fluid leakage can occur from natural orifices such as the anus and vagina, spontaneously formed enteric fistulas, and surgical openings such as ileostomy and colostomy stomas. The volume of leakage from a high-output fistula or stoma can range from two to ten or more liters per day, which makes it difficult or impossible for patients to leave their bed and can lengthen hospital stays by weeks or months. In many instances, the leaked fluid causes skin irritation, infection, pain, and feelings of isolation and depression for the patient. Management of such uncontrolled leakage can present many challenges for the patients and healthcare professionals.

A wide range of pouch appliances are available to capture and contain effluent from ostomies and other discharge sites. Example devices that may be used to contain the fluids exiting the body of a patient are described in International Patent Publication No. WO 2011/015203 A1 to Weldingh, International Patent Publication No. WO 2004/0844778 A2 to Ciok, U.S. Patent Publication No. 2008/0269700 A1 to O'Toole, U.S. Patent No. 5,429,626 to Fenton, and U.S. Patent No. 5,015,244 to Cross, all of which are incorporated herein by reference in their entireties.

Such devices, however, tend to rely solely on adhesive materials such as hydrocolloids to remain affixed to the patient's skin around the perimeter of the fistula or orifice. These materials do not adhere well when the patient's skin is irritated, weeping, or exuding fluid, uneven due to wounds or scarring, in a moist area of the body, or when they are exposed to constant flows of high-volume effluent. Even with proper placement of such pouch appliances fluid leakage can still occur. There remains a need for an improved containment system.

### SUMMARY

This disclosure describes embodiments of a containment system that may be used to contain fluid leakage from openings in the body of a patient. As mentioned above, fluid leakage can occur from natural orifices such as the anus and vagina, spontaneously formed enteric fistulas, rectovaginal fistulas, and surgical openings such as ileostomy and colostomy stomas. As described further below, the components are intended to be used together as a system to address leakage problems that are commonly observed with conventional pouch appliances.

Embodiments are directed to a system which can include a vacuum-adherent pouch device comprising a pouch connected to a porous foam ring which has a skin-safe air-permeable contact layer on its bottom surface and an air seal on its top surface. The inner and outer perimeters of the foam ring are enclosed and sealed with skin-safe adhesive materials. When placed on the patient, negative pressure (i.e. vacuum or near vacuum) is applied to the foam ring, causing the foam to collapse and compress the vacuum-adherent pouch device against the patient's skin forming a seal around the perimeter of the fistula or orifice. Negative pressure in combination with the adhesive materials on the perimeters of the foam ring form a fluid barrier around the opening in the body of the patient so that leakage can be directed into the pouch and drawn away to a separate effluent container, or simply emptied into a toilet.

One embodiment includes a fluid containment system for use in collecting and containing effluent from a bodily orifice. The fluid containment system includes a vacuum-adherent pouch device. The vacuum-adherent pouch device includes a vacuum-adherent base. The vacuum-adherent base further includes a foam wall defining an opening. The foam wall further includes an air permeable lower surface. The foam wall also includes a sealing layer coupled to top and side portions of the foam wall. The sealing layer can form an inner sealing surface and an outer sealing surface. The inner sealing surface and the outer sealing surface can be substantially coplanar with the air permeable lower surface. The inner sealing surface can be formed within the opening of the foam wall and the outer sealing surface formed outside the foam wall. The vacuum-adherent pouch device also includes a pouch configured to collect fluids. The pouch can also include a drain and be coupled to the sealing layer at the inner sealing surface. The vacuum-adherent pouch device also includes a negative pressure coupling configured to fluidly couple a vacuum source to the foam wall. The fluid containment system further includes a negative pressure pump having one or more ports. One of the one or more ports can be coupled to the negative pressure coupling. The fluid containment system further includes an effluent container having one or more effluent ports with one of the one or more effluent ports coupled to the drain.

In one embodiment, the negative pressure pump includes a first port coupled to the negative pressure coupling and a second port coupled to one of the one or more effluent ports of the pouch.

In one embodiment, the effluent container includes a first effluent port coupled to the drain of the pouch and a second effluent port coupled to a vacuum source. The second effluent port can be coupled to a central hospital vacuum line. The second effluent port can be coupled to one of the one or more ports of the negative pressure pump.

In one embodiment, the foam wall of the vacuum-adherent pouch device comprises reticulated foam.

In one embodiment the foam wall forms a ring shape. The foam wall forms a ring shape with an external protrusion or bridge. In this embodiment, the negative pressure coupling fluidly couples to the external protrusion.

In one embodiment, the inner surface and the outer surface of the vacuum-adherent pouch device include a skin adherent. In yet another embodiment, the inner surface of the vacuum-adherent pouch device includes an aperture sized to surround the bodily orifice. The inner surface can be selectively puncturable.

In one embodiment, the air permeable lower surface of the foam wall includes an air permeable skin contact layer.

In embodiments, a tube channel can be formed through one or more portions of the sealing layer and the foam wall.

The above summary is not intended to describe each illustrated embodiment or every implementation of the subject matter hereof. The figures and the detailed description that follow more particularly exemplify various embodiments.

### BRIEF DESCRIPTION OF THE DRAWINGS

Subject matter hereof may be more completely understood in consideration of the following detailed description of various embodiments in connection with the accompanying figures, in which:
FIGS. 1 is a side view of an example of a vacuum-adherent pouch system, according to embodiments described herein.
FIG. 2 is a side view of a wearable example of a vacuum-adherent pouch system, according to embodiments described herein.
FIG. 3 is a side view of a wearable example of a vacuum-adherent pouch system, according to embodiments described herein.
FIG. 4A is a top view of an example vacuum-adherent pouch device, according to embodiments described herein.
FIG. 4B is a bottom view of an example vacuum-adherent pouch device, according to embodiments described herein.
FIG. 4C is a bottom view of an example vacuum-adherent pouch device after it has been prepared for application on a patient, according to embodiments described herein.
FIG. 4D is a top view of an example vacuum-adherent pouch device after it has been placed on a patient, according to embodiments described herein.
FIG. 5A is a cross-sectional view of the example vacuum-adherent pouch device shown in FIG. 4A, according to embodiments described herein.
FIG. 5B is a cross-sectional view of the example vacuum-adherent pouch device shown in FIG. 4D, according to embodiments described herein.
FIG 5C is an exploded view of the example vacuum-adherent pouch device shown in FIG. 5A showing device subcomponent options, according to embodiments described herein.
FIGS. 6A and 6B are cross-section views of an example vacuum-adherent pouch device illustrating example options for passing fistuloclysis and other tubes through the vacuum-adherent pouch device, according to embodiments described herein.
FIGS. 7A and 7B are top-views of alternative embodiments of a vacuum-adherent pouch device, according to embodiments described herein.
FIG. 8 is an isometric view of an example effluent container, according to embodiments described herein.

While various embodiments are amenable to various modifications and alternative forms, specifics thereof have been shown by way of example in the drawings and will be described in detail. It should be understood, however, that the intention is not to limit the claimed inventions to the particular embodiments described. On the contrary, the intention is to cover all modifications, equivalents, and alternatives falling within the scope of the subject matter as defined by the claims.

### DETAILED DESCRIPTION OF THE DRAWINGS

The present disclosure describes embodiments of a containment system that can be used to contain fluid leakage (e.g., gastric fluid, stool, urine, wound drainage, and the like) from openings in the body of a patient. Fluid leakage can occur from natural orifices such as the anus and vagina, spontaneously formed enteric fistulas, rectovaginal fistulas, and surgical openings such as ileostomy and colostomy stomas. As described in further detail below, the components are intended to be used together as a system to address one or more of the problems outlined above. By way of example, the system components disclosed herein may be used to at least one of create a fluid barrier (e.g., a seal) between the vacuum-adherent pouch device and the skin of the patient, redirect any fluid leakage away from the skin of the patient, and draw leakage to an effluent container. The fluid barrier may substantially or completely prevent the passage of fluid leaking through the points of contact between the vacuum-adherent pouch device and the body of the patient.

Unless otherwise indicated, all numbers expressing feature sizes, amounts, and physical properties are to be understood as being modified by the term "about." Accordingly, unless indicated to the contrary, the numerical parameters set forth are approximations that can vary depending upon the desired properties sought to be obtained by those skilled in the art utilizing the teachings disclosed herein.

As used herein, the singular forms "a", "an", and "the" encompass embodiments having plural referents, unless the content clearly dictates otherwise. As used in this specification and the appended claims, the term "or" is generally employed in its sense including "and/or" unless the content clearly dictates otherwise.

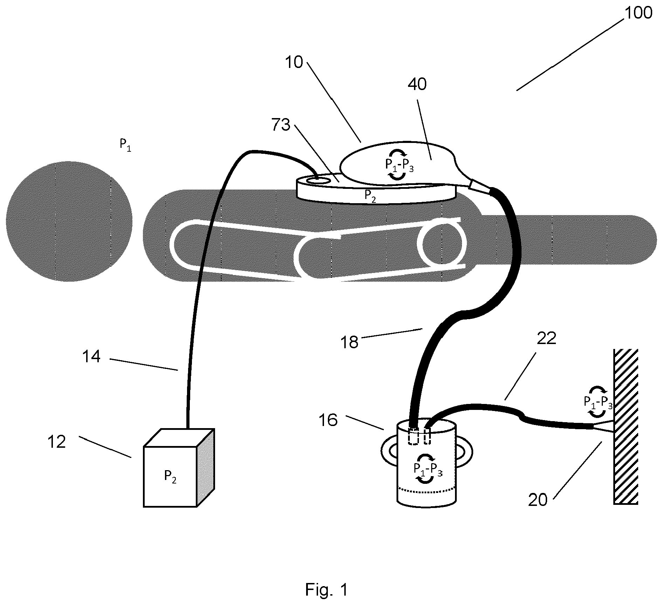

FIG. 1 depicts an example containment system 100 that is placed on a patient. Containment system 100 includes a vacuum-adherent pouch device 10 connected by an air tube 14 to a negative pressure pump 12. In embodiments, vacuum-adherent pouch device 10 is configured for placement and applied to skin of the patient around the enteric fistula, ostomy, vagina, anus, or other body opening. Vacuum-adherent pouch device 10 also includes a base 73 and a pouch 40.

The pump 12 creates a vacuum pressure P₂ less than atmospheric pressure P₁ in the base 73 of the vacuum-adherent pouch device 10 to affix it against the patient's skin to create a fluid barrier (e.g., a seal) between the vacuum-adherent pouch device 10 and the skin. The pouch 40 of the vacuum-adherent pouch device 10 is also connected to an effluent container 16 by an effluent drain tube 18. The effluent container 16 is connected by an air tube 22 to hospital wall suction 20. Hospital wall suction 20 is applied intermittently at a pressure P₃ less than atmospheric pressure P₁ to the effluent container 16 to draw accumulated leakage from the vacuum-adherent pouch device 10 through the effluent drain tube 18 and into the effluent container 16. The design of the effluent container 16 combined with intermittent/cycling of negative pressure allows effluent to be drawn into and retained in the effluent container 16 without fouling the hospital wall suction system 20.

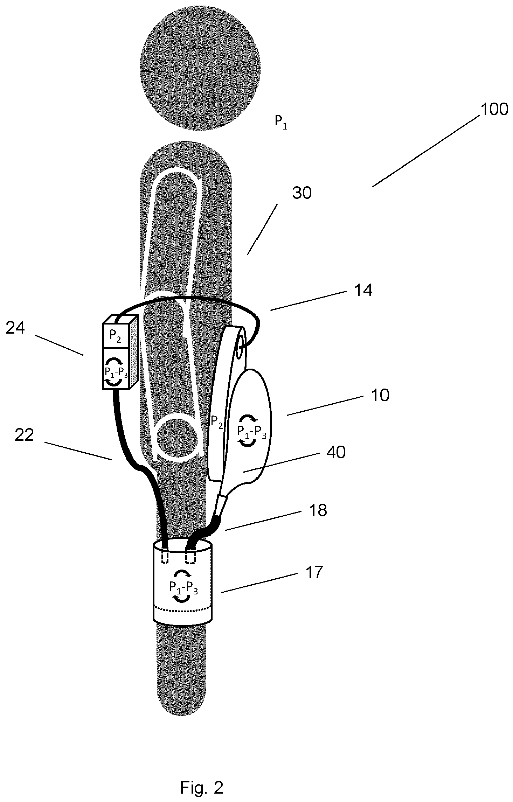

As depicted in FIG. 2, a wearable example of the containment system 100 is placed on a patient. Containment system 100 includes a vacuum-adherent pouch device 10 connected by air tube 14 to a combination negative pressure and intermittent suction pump 24. Pump 24 creates a vacuum pressure P₂ less than atmospheric pressure P₁ in base 73 of the vacuum-adherent pouch device 10 to affix it against the patient's skin to create a fluid barrier (e.g., a seal) between the vacuum-adherent pouch device 10 and the skin. The pouch 40 of the vacuum-adherent pouch device 10 is also connected to a wearable effluent container 17 by effluent drain tube 18. The effluent container 17 is connected by air tube 22 to pump 24. Pump 24 applies suction intermittently at a pressure P₃ less than atmospheric pressure P₁ to the effluent container 17 to draw accumulated leakage from the vacuum-adherent pouch device 10 through the effluent drain tube 18 and into the effluent container 17. The design of the effluent container 17 combined with intermittent/cycling of negative pressure allows effluent to be drawn into and retained in the effluent container 17 without fouling the pump 24.

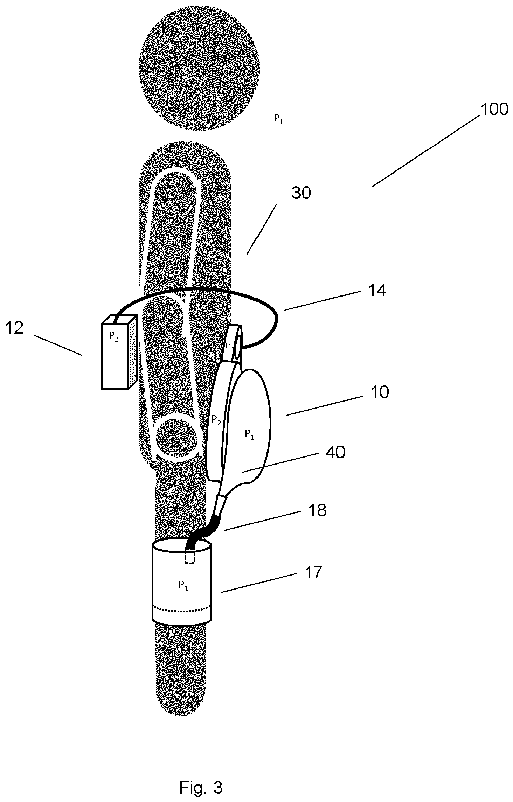

FIG. 3 illustrates another wearable example of the containment system 100 that is placed on a patient. The system includes vacuum-adherent pouch device 10 connected by air tube 14 to negative pressure pump 12. The pump 12 creates a vacuum pressure P₂ less than atmospheric pressure P₁ in the base of the vacuum-adherent pouch device 10 to affix it against the patient's skin to create a fluid barrier (e.g., a seal) between the vacuum-adherent pouch device 10 and the skin. Pouch 40 of the vacuum-adherent pouch device 10 is also connected to wearable effluent container 17 by an effluent drain tube 18. Accumulated leakage from the vacuum-adherent pouch device 10 drains by gravity through the effluent drain tube 18 and into the effluent container 17.

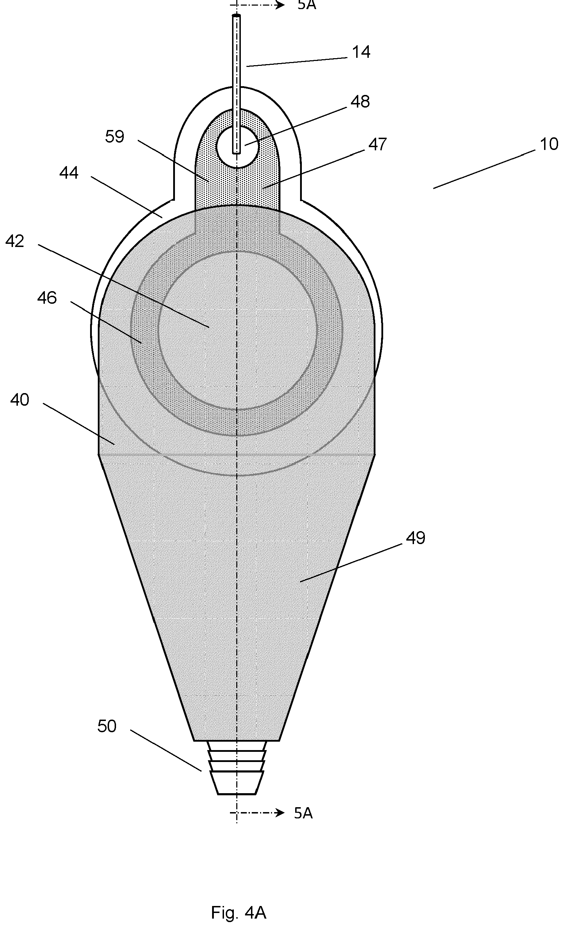

FIG. 4A depicts a top view of an embodiment of vacuum-adherent pouch device 10. Vacuum-adherent pouch device 10 includes pouch 40, a reticulated open cell foam ring 46, a reticulated open cell foam bridge 59, a film or other air impermeable material 47 on the top surface of the foam ring 46 and foam bridge 59, a skin-adhesive bottom 42, a skin-adhesive exterior perimeter 44, a negative pressure connector 48, negative pressure air tube 14, a fluid-capture space 49, and an effluent drain connector 50.

The foam bridge 59 is connected to the foam ring 46. The pouch 40 is connected to the foam ring 46. The pouch 40 and the interior perimeter of the foam ring 46 are sealed to the skin-adhesive bottom 42 to form an enclosed fluid-capture space 49. The exterior perimeter of the foam ring 46 and foam bridge 59 are enclosed and sealed with skin-adhesive material 44. The top surface of the foam ring 46 and foam bridge 59 are sealed with a film or other air impermeable material 47 which has a negative pressure connector 48 and air tube 14 attached to supply negative pressure to the foam bridge 59.

To prepare the vacuum-adherent pouch device 10 for application on a patient, a hole is cut in the skin-adhesive bottom 42, sized to fit around the enteric fistula, ostomy, vagina, or other body opening. When the vacuum-adherent pouch device 10 is placed on a patient, the skin-adhesive bottom 42 and the skin-adhesive exterior perimeter 44 form concentric seals between the interior and exterior perimeters of the foam ring 46 and the skin of the patient. Negative pressure is then supplied to the foam ring 46 via the foam bridge 59 and air tube 14 and negative pressure connector 48 to compress the foam ring 46 and affix the vacuum-adherent pouch device 10 to the patient. Fluid leakage is then redirected to the fluid-capture space 49 where it can then be drawn through the effluent drain connector 50 to an effluent container.

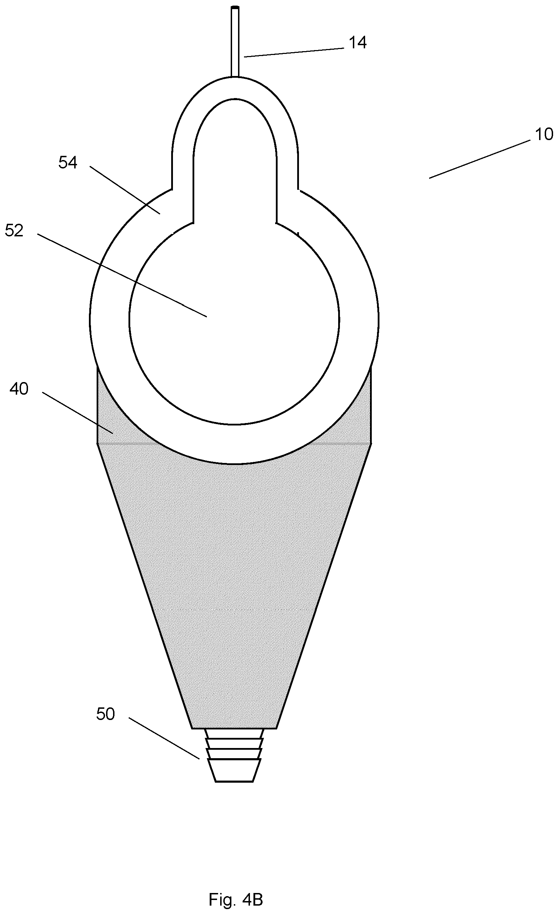

FIG 4B depicts a bottom view of an example vacuum-adherent pouch device 10. Vacuum-adherent pouch device 10 includes release paper 52 and release paper 54. Release paper 52 is configured to protect the contact layer and skin-adhesive bottom. Release paper 54 is configured to protect the skin-adhesive exterior perimeter.

To prepare the vacuum-adherent pouch device 10 for application on a patient, a hole is cut through release paper 52 and the skin-adhesive bottom, sized to fit around the enteric fistula, ostomy, vagina, or other body opening. Before the vacuum-adherent pouch device 10 is placed on a patient, release paper 52 is removed to expose the contact layer and skin-adhesive bottom and release paper 54 is removed to expose the skin-adhesive exterior perimeter.

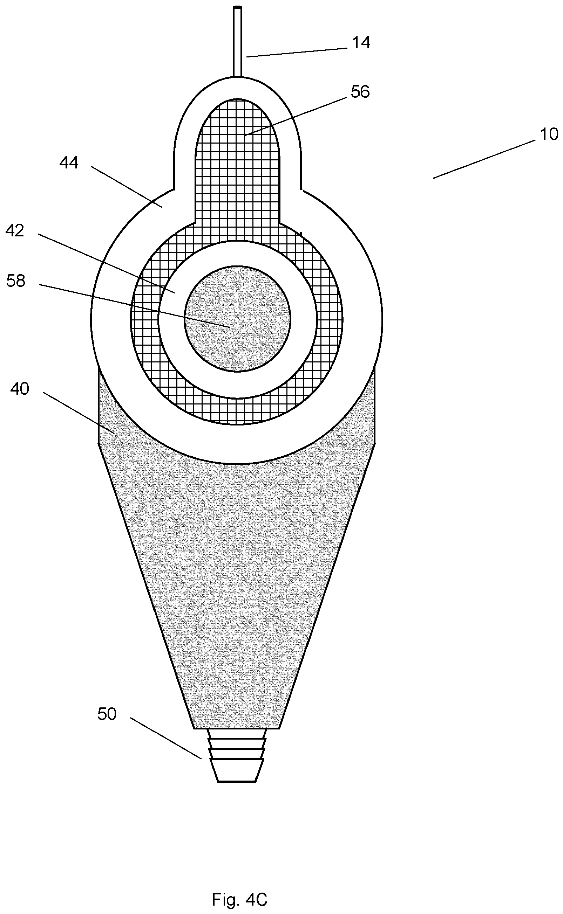

FIG. 4C is the bottom view of an example vacuum-adherent pouch device 10 after it has been prepared for application on a patient and includes the skin-adhesive exterior perimeter 44, the air-permeable contact layer 56 on the base of the reticulated open cell foam ring 46 and foam bridge 59, the skin-adhesive bottom 42, and the hole 58 that has been cut in the skin-adhesive bottom 42.

When the vacuum-adherent pouch device 10 is placed on a patient, the hole 58 that has been cut in the skin-adhesive bottom 42 fits around the enteric fistula, ostomy, vagina, or other body opening. The skin-adhesive exterior perimeter 44 and the uncut portion of the skin-adhesive bottom 42 form concentric seals on the skin of the patient. The air-permeable contact layer 56 rests on the skin between concentric skin-adhesives 42 and 44 and protects the skin when negative pressure is supplied to compress the foam ring and foam bridge and affix the vacuum-adherent pouch device 10 to the patient.

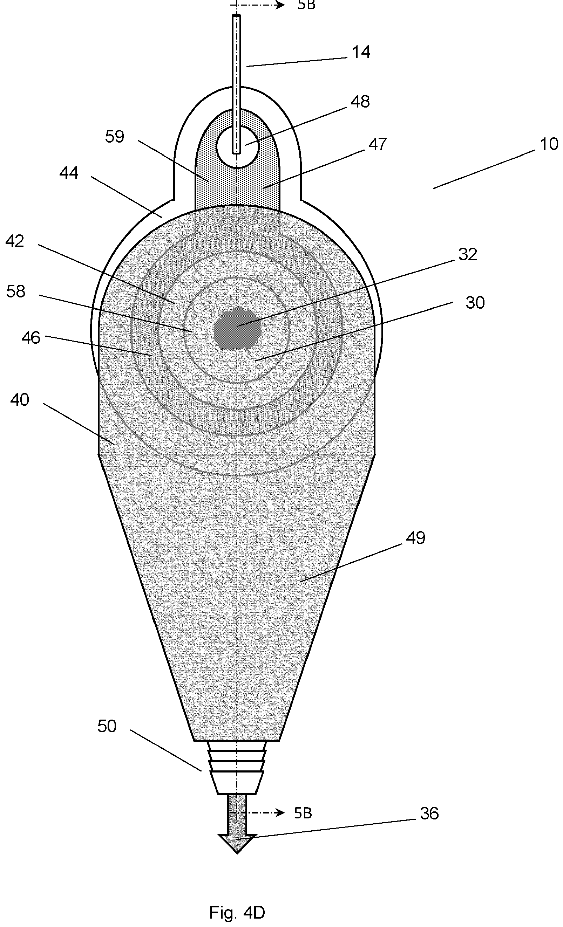

FIG. 4D is a top view of an example vacuum-adherent pouch device 10 after it has been placed on a patient and includes the patient's skin 30, an enteric fistula, ostomy, vagina, or other body opening 32, the pouch 40, the reticulated open cell foam ring 46, the reticulated open cell foam bridge 59, the film or other air impermeable material 47 on the top surface of the foam ring 46 and the foam bridge 59, the skin-adhesive bottom 42, the hole 58 that has been cut in the skin-adhesive bottom 42, the skin-adhesive exterior perimeter 44, the negative pressure connector 48, the negative pressure air tube 14, the fluid-capture space 49, the effluent drain connector 50, and accumulated leakage 36 being drawn from fluid-capture space 49 to an effluent container.

When the vacuum-adherent pouch device 10 is placed on a patient, the hole 58 cut in the skin-adhesive bottom 42 fits around the enteric fistula, ostomy, vagina, or other body opening 32. The skin-adhesive exterior perimeter 44 and the uncut portion of the skin-adhesive bottom 42 form concentric seals on the skin of the patient. Negative pressure is supplied to the foam ring 46 via the foam bridge 59 and air tube 14 and negative pressure connector 48 to compress the film or other air impermeable material 47 and the foam ring 46 and affix the vacuum-adherent pouch device 10 to the patient. Fluid leakage 36 is then redirected to the fluid-capture space 49 where it can then be drawn through the effluent drain connector 50 to an effluent container.

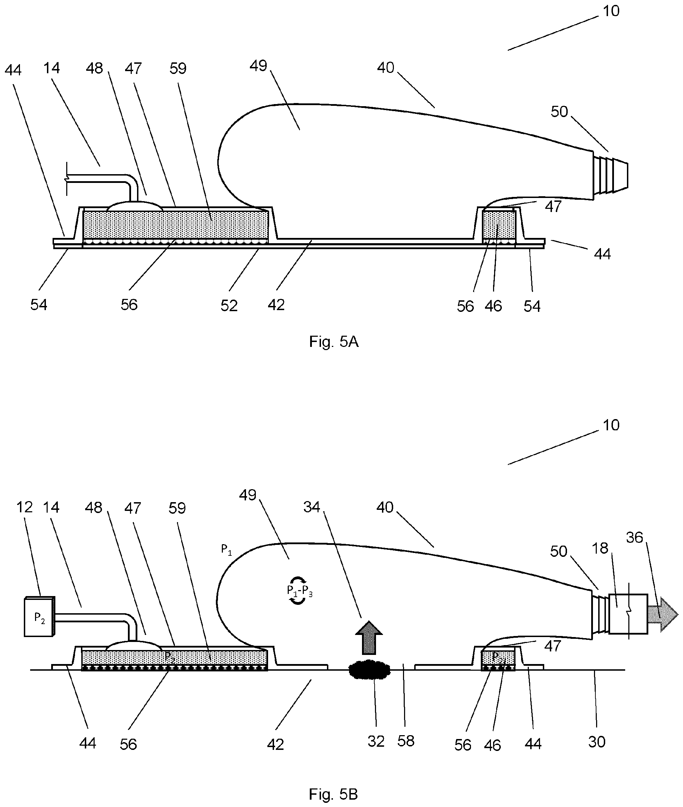

FIG. 5A is a cross-sectional view of the example vacuum-adherent pouch device 10 shown in FIG. 4A and includes the pouch 40, the reticulated open cell foam ring 46, the reticulated open cell foam bridge 59, the film or other air impermeable material 47 on the top surface of the foam ring 46 and foam bridge 59, the air-permeable contact layer 56 on the base of the foam ring 46 and foam bridge 59, the skin-adhesive bottom 42, the skin-adhesive exterior perimeter 44, release paper 52 protecting the contact layer and skin-adhesive base 42, release paper 54 protecting the skin-adhesive exterior perimeter, a negative pressure connector 48, a negative pressure air tube 14, the fluid-capture space 49, and the effluent drain connector 50.

The pouch 40 is connected to the foam ring 46. The pouch 40 and the interior perimeter of the foam ring 46 are sealed to the skin-adhesive bottom 42 to form an enclosed fluid-capture space 49. The exterior perimeter of the foam ring 46 and foam bridge 59 are enclosed and sealed with skin-adhesive material 44. The top surface of the foam ring 46 and foam bridge 59 are sealed with a film or other air impermeable material 47 which has a negative pressure connector 48 and air tube 14 attached to supply negative pressure to the foam ring 46. The air-permeable contact layer 56 covers the base of the foam ring 46 and foam bridge 59 and affixes vacuum-adherent pouch device 10 to the patient and also protects the skin when negative pressure is supplied.

Any suitable material may be used to form all or part vacuum-adherent pouch device 10, as will be recognized by those skilled in the art. In some examples, pouch 40 may be formed from one or more biocompatible, sterilizable, materials including, for example, plastics or rubbers. In some examples, foam ring 46 may be formed from one or more biocompatible, sterilizable, materials including, for example, reticulated open cell foam. In some examples, film or other air impermeable material 47 may be formed from one or more biocompatible, sterilizable, materials including, for example, adhesive plastic drape or film. In some examples, air-permeable contact layer 56 may be formed from one or more biocompatible, sterilizable, materials including, for example, non-adherent films, plastics, rubbers, silicones, polyester or other fabrics with pores, slits, or porosities that allow the passage of air. In some examples, skin-adhesive bottom 42 and skin-adhesive exterior perimeter 44 may be formed from one or more biocompatible, sterilizable, materials including, for example, hydrocolloids, silicones, or adhesive plastic drapes or films.

In some examples, vacuum-adherent pouch device 10 may be sterilized for use in a surgical environment. Additionally, or alternatively vacuum-adherent pouch device 10 may be a component of containment system kit that includes vacuum-adherent pouch device 10 which may be sterilized and packaged in a sealed pouch or container that preserves sterility and can be opened by the patient or clinician.

FIG. 5B is a cross-sectional view of the example vacuum-adherent pouch device 10 shown in FIG. 4D after it has been placed on a patient and includes the patient's skin 30, an enteric fistula, ostomy, vagina, or other body opening 32, the pouch 40, the reticulated open cell foam ring 46, the reticulated open cell foam bridge 59, the film or other air impermeable material 47 on the top surface of the foam ring 46 and foam bridge 59, the air-permeable contact layer 56 on the base of the foam ring 46 and foam bridge 59, the skin-adhesive bottom 42, the hole 58 that has been cut in the skin-adhesive bottom 42, the skin-adhesive exterior perimeter 44, the negative pressure connector 48, the negative pressure air tube 14, the negative pressure pump 12, the fluid-capture space 49, intestinal and other bodily fluid effluent 34, an effluent drain connector 50, the effluent drain tube 18, and accumulated leakage 36 being drawn from fluid-capture space 49 to an effluent container.

When the vacuum-adherent pouch device 10 is placed on a patient, the hole 58 is cut in the skin-adhesive bottom 42 to fit around the enteric fistula, ostomy, vagina, or other body opening 32. The skin-adhesive exterior perimeter 44 and the uncut portion of the skin-adhesive bottom 42 form concentric seals on the skin of the patient. Negative pressure is supplied to the foam ring 46 from the negative pressure pump 12 via foam bridge 59 and the air tube 14 and negative pressure connector 48 to compress the foam ring 46 and the surface film or other air impermeable material 47. The air-permeable contact layer 56 covers the base of the foam ring 46 and foam bridge 59 and affixes vacuum-adherent pouch device 10 to the patient and also protects the skin. Intestinal and other bodily fluid effluent 34 from the enteric fistula, ostomy, vagina, or other body opening is directed to the fluid-capture space 49. Accumulated leakage 36 is drawn through the effluent drain connector 50 and effluent drain tube 18 to an effluent container.

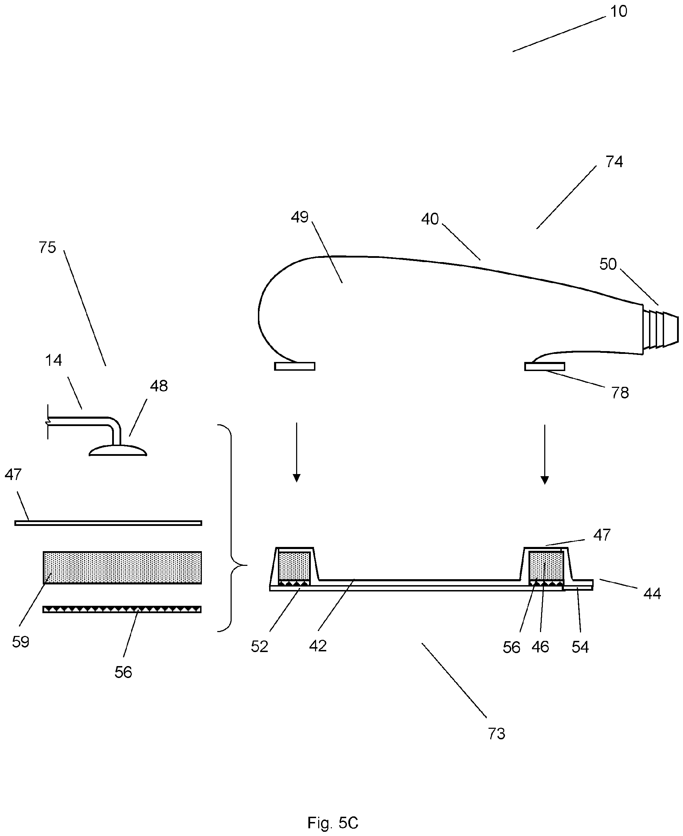

FIG 5C is a view of the example vacuum-adherent pouch device shown in FIG. 5A showing device subcomponent options. Vacuum-adherent pouch device 10 may be provided for use in whole or as subcomponents for assembly at the bedside as will be recognized by those skilled in the art. For example, vacuum-adherent base subcomponent 73 and pouch subcomponent 74 may be fastened together with an adhesive ring 78. A bridge to provide negative pressure to vacuum-adherent base subcomponent 73 may be formed by assembling air-permeable skin contact layer 56, the reticulated open cell foam bridge 59, film or other air impermeable material 47, and negative pressure attachment subcomponent 75. These or other subcomponents may be assembled when the vacuum-adherent pouch device 10 is placed on the patient.

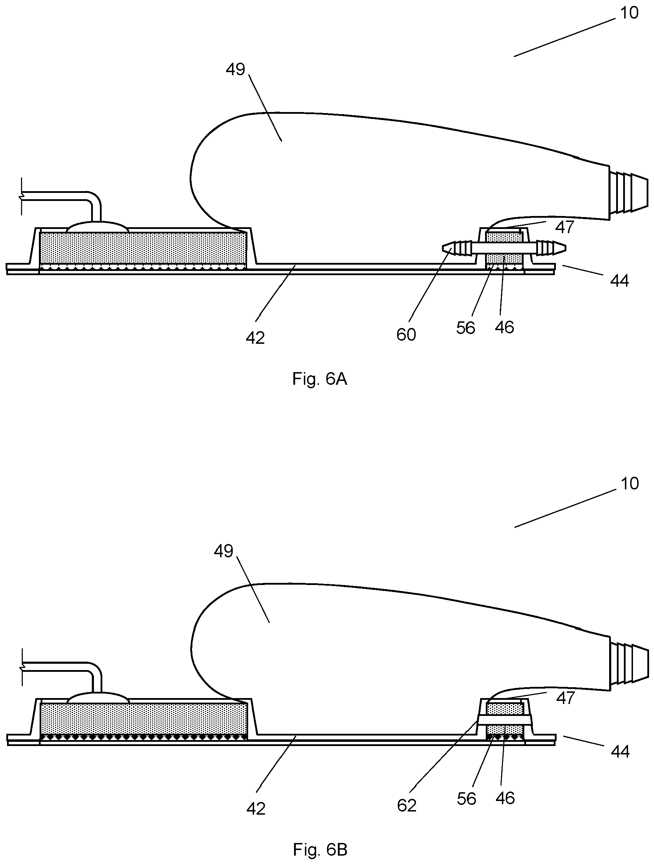

FIGS. 6A and 6B show examples of a vacuum-adherent pouch device 10 that include an optional feature to enable passage of fistuloclysis (fistula feeding), catheter, and other tubes for the purpose of transporting fluids into or out of the enteric fistula, ostomy, vagina, or other body opening. FIG. 6A illustrates a cross-sectional view of an example vacuum-adherent pouch device 10 with a tube connector 60 for splicing a tube into the fluid-capture space 49. FIG. 6B illustrates a cross-sectional view of an example vacuum-adherent pouch device 10 with a tube channel 62 for passing a tube into the fluid-capture space 49. Preferably, tube connector 60 and tube channel 62 are formed using a non-fluid permeable and/or non-porous flexible material. Further, as will be recognized by those skilled in the art, the tube connector 60 and tube channel 62 can be sized and shaped to accommodate all different sizes and shapes medical tubes.

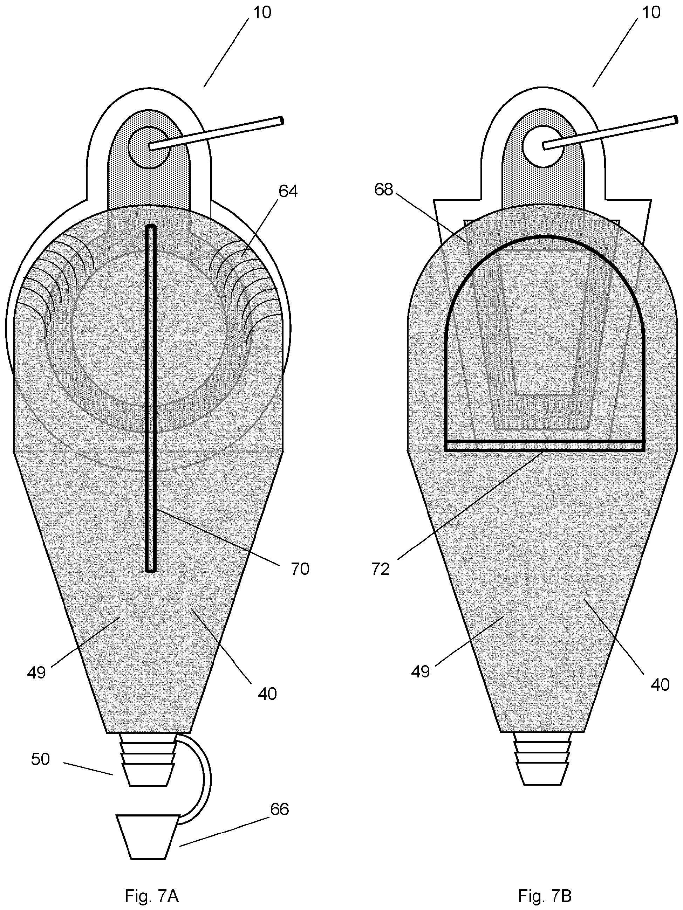

The vacuum-adherent pouch device 10 may include any or all of the following optional features shown and described with respect to the additional examples of FIGS. 7A and 7B. The features disclosed and described in the context of FIGS. 4A to 6B may be used in combination with any of the other features described herein such that it will be understood that any feature or specific combination of features shown in the accompanying figures is not intended to be limited to only the specific embodiments illustrated.

The example vacuum-adherent pouch device 10 illustrated in FIG. 7A includes bellows or pleats 64 in the pouch 40 to enable expansion of the vacuum-adherent pouch device 10 to fit larger body openings, an effluent drain connector cap 66 which is integral to the effluent drain connector 50 and snaps onto the effluent drain connector 50 to seal the fluid-capture space 49, and a resealable zip-type opening 70 to access the fluid-capture space 49.

The example vacuum-adherent pouch device 10 illustrated in FIG. 7B includes a resealable lid opening 72 to access the fluid-capture space 49, and an alternative form factor 68 to fit the perineal area. Further, as will be recognized by those skilled in the art, the vacuum-adherent pouch device 10 can be sized and shaped to accommodate all different sizes and shapes of patients and body openings.

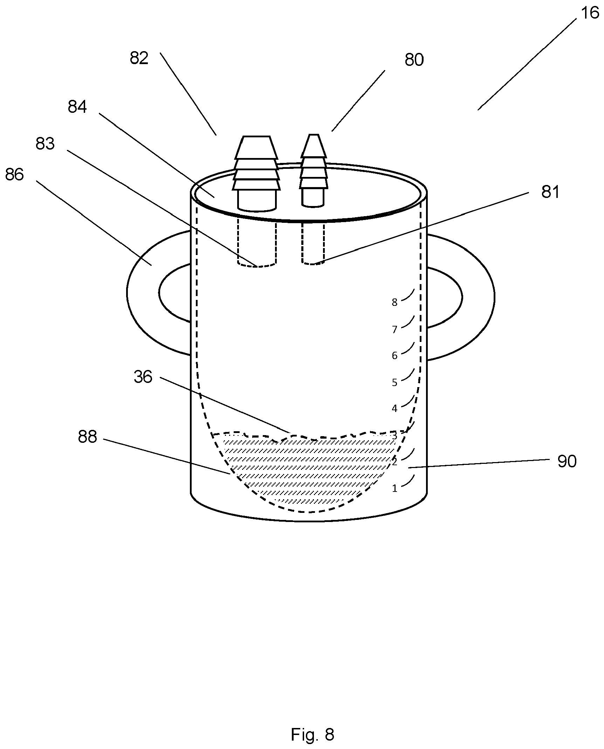

FIG. 8 illustrates an example effluent container 16 and includes an intermittent negative pressure connector 80 with a vacuum port 81 inside the container 16, an effluent container connector 82 with an effluent port 83 inside the container 16, a resealable lid 84, carrying handle(s) 86, a water proof liner 88, and fluid measurement marks 90 on the exterior of the effluent container 16.

When the containment system is placed on a patient, the effluent container 16 is connected to intermittent suction via the intermittent negative pressure connector 80, and to the vacuum-adherent pouch device via the effluent container connector 82. When intermittent suction is applied to the effluent container 16, negative pressure draws accumulated leakage 36 from the vacuum-adherent pouch device into the effluent container 16 through the effluent port 83.

The effluent port 83 and the vacuum port 81 are separated from each other near the top of the container 16 so that leakage 36 that is drawn into the effluent container 16 falls to the bottom of the container 16. The separation of the effluent port 83 from the vacuum port 81 and the application of intermittent or cycling negative pressure prevents effluent from being drawn into the vacuum port 81 where it may foul the intermittent negative pressure pump.

The effluent container 16 may include any or all of the following optional features including a resealable lid 84 for use in dumping the effluent container 16 when full of leakage 36, carrying handle(s) 86 for use in carrying the effluent container 16, a disposable water proof liner 88 as an alternative to dumping and cleaning the effluent container 16, and fluid measurement marks 90 on the walls of the effluent container 16 to measure the output of enteric fistulas, rectovaginal fistulas, and ostomy stomas.

Preferably, effluent container 16 is formed using a non-fluid permeable and/or non-porous flexible material which is transparent or translucent so the volume level of accumulated leakage 36 can be seen through the walls of the effluent container 16. Further, as will be recognized by those skilled in the art, the effluent container 16 can be sized and shaped to accommodate all different effluent volumes and use scenarios to include hospital, in-home, and wearable.

Various embodiments of systems, devices, and methods have been described herein. These embodiments are given only by way of example and are not intended to limit the scope of the claimed inventions. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the claimed inventions.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

Although a dependent claim may refer in the claims to a specific combination with one or more other claims, other embodiments can also include a combination of the dependent claim with the subject matter of each other dependent claim or a combination of one or more features with other dependent or independent claims. Such combinations are proposed herein unless it is stated that a specific combination is not intended.

Any incorporation by reference of documents above is limited such that no subject matter is incorporated that is contrary to the explicit disclosure herein. Any incorporation by reference of documents above is further limited such that no claims included in the documents are incorporated by reference herein. Any incorporation by reference of documents above is yet further limited such that any definitions provided in the documents are not incorporated by reference herein unless expressly included herein.

## Claims

1. A vacuum-adherent pouch device for use in a fluid containment system for use in collecting and containing effluent from a bodily orifice, the vacuum-adherent pouch device comprising:
a vacuum-adherent base including:
a wall including structure defining an opening, the wall further having an air permeable lower surface, and
a sealing layer coupled to top and side portions of the wall, the sealing layer forming an inner sealing surface and an outer sealing surface, the inner sealing surface and the outer sealing surface being substantially coplanar with the air permeable lower surface, the inner sealing surface formed within the opening of the wall and the outer sealing surface formed outside the wall;
a pouch configured to collect fluids, the pouch further including a drain, the pouch coupled to the sealing layer at the inner sealing surface; and
a negative pressure coupling configured to fluidly couple a vacuum source to the wall.

2. The vacuum-adherent pouch device of claim 1, wherein the wall comprises reticulated foam.

3. The vacuum-adherent pouch device of claim 1 or claim 2, wherein the wall forms a ring shape, and preferably wherein the wall forms a ring shape with an external protrusion, and preferably wherein the negative pressure coupling fluidly couples to the external protrusion.

4. The vacuum-adherent pouch device of claim 1, claim 2 or claim 3, wherein the inner surface and the outer surface include a skin adherent.

5. The vacuum-adherent pouch device of claim 1 or any preceding claim, wherein the inner surface includes an aperture sized to surround the bodily orifice.

6. The vacuum-adherent pouch device of any preceding claim, wherein the inner surface is selectively puncturable.

7. The vacuum-adherent pouch device of claim 1 or any preceding claim, wherein the air permeable lower surface includes an air permeable skin contact layer.

8. The vacuum-adherent pouch device of claim 1 or any preceding claim, wherein a tube channel is formed through one or more portions of the sealing layer and the wall.

9. A fluid containment system for use in collecting and containing effluent from a bodily orifice, the fluid containment system comprising:
a vacuum-adherent pouch device, including:
a vacuum-adherent base further including:
a foam wall defining an opening, the foam wall further having an air permeable lower surface; and
a sealing layer coupled to top and side portions of the foam wall, the sealing layer forming an inner sealing surface and an outer sealing surface, the inner sealing surface and the outer sealing surface being substantially coplanar with the air permeable lower surface, the inner sealing surface formed within the opening of the foam wall and the outer sealing surface formed outside the foam wall,
a pouch configured to collect fluids, the pouch further including a drain, the pouch coupled to the sealing layer at the inner sealing surface, and
a negative pressure coupling configured to fluidly couple a vacuum source to the foam wall;
a negative pressure pump having one or more ports, one of the one or more ports coupled to the negative pressure coupling; and
an effluent container having one or more effluent ports, one of the one or more effluent ports coupled to the drain.

10. The system of claim 9, wherein the negative pressure pump includes a first port coupled to the negative pressure coupling and a second port coupled to one of the one or more effluent ports of the effluent container.

11. The system of claim 9 or 10, wherein the effluent container includes a first effluent port coupled to the drain of the pouch and a second effluent port coupled to a vacuum source and preferably wherein the second effluent port is coupled to a central hospital vacuum line or wherein the second effluent port is coupled to one of the one or more ports of the negative pressure pump.

12. The system of any of claims 9 to 11, wherein the foam wall forms a ring shape with an external protrusion and preferably wherein the negative pressure coupling fluidly couples to the external protrusion.

13. The system of any of claims 9 to 12, wherein the inner surface and the outer surface include a skin adherent.

14. The system of any of claims 9 to 13, wherein the inner surface includes an aperture sized to surround the bodily orifice.

15. The system of any of claims 9 to 14, wherein a tube channel is formed through one or more portions of the sealing layer and the foam wall.
